# EUROPEAN PATENT SPECIFICATION

(11) **EP 3 595 467 B1**
(45) Date of publication and mention of the grant of the patent: **19.05.2021**
(21) Application number: 18715501.5
(22) Date of filing: 14.03.2018
(51) Int. Cl.: A24F 40/46

(54) **A MOUTHPIECE AND HEATER ASSEMBLY FOR AN INHALATION DEVICE**
MUNDSTÜCK UND HEIZANORDNUNG FÜR EINE INHALATIONSVORRICHTUNG
ENSEMBLE EMBOUT BUCCAL ET DISPOSITIF DE CHAUFFAGE POUR UN DISPOSITIF D'INHALATION

(30) Priority: 16.03.2017 GB 201704167; 26.05.2017 GB 201708472; 20.06.2017 GB 201709864
(43) Date of publication of application: 22.01.2020
(73) Proprietor: Ventus Medical Limited, Liverpool L19 2RF (GB)
(72) Inventor: CANE, Michael, Cambridge Cambridgeshire CB23 2RF (GB); HART, Oliver, Cambridge Cambridgeshire CB23 2RF (GB); DIGNUM, Mark, Liverpool L19 2RF (GB); LAWSON, David, Liverpool L19 2RF (GB)
(74) Representative: Denmark, James Christopher
(86) International application number: PCT/EP2018/056429
(87) International publication number: WO 2018/167166

(56) References cited:
- WO-A1-2016/005530
- WO-A1-2016/005533
- WO-A1-2016/162446
- US-A1- 2013 255 702
- US-A1- 2014 060 554

## Description

### Technical Field

The present invention relates to a mouthpiece and Heater assembly for an Inhalation Device. The heater is configured to heat a composition to generate an aerosol for inhalation by a user. In particular, but not exclusively, the present invention relates to a heater for a nicotine replacement therapy or a smoking-substitute device. Furthermore, the present invention relates to a mouthpiece comprising the heater, an inhalation device comprising the heater and a method of manufacturing the heater.
Although the present application will focus on heating compositions containing nicotine for inhalation by user, it will be appreciated that the heater can be used for heating compositions comprising other compounds, for example, medicaments or flavourings.

### Background

Nicotine replacement therapies are aimed at people who wish to stop smoking and overcome their dependence on nicotine. One form of nicotine replacement therapy is an inhaler or inhalator, one example of which is sold by Johnson & Johnson Limited under the brand name Nicorette®. These generally have the appearance of a plastic cigarette and are used by people who crave the behaviour associated with consumption of combustible tobacco - the so-called hand-to-mouth aspect - of smoking tobacco. An inhalator comprises a replaceable nicotine cartridge. When a user inhales through the device, nicotine is atomised from the cartridge and is absorbed through the mucous membranes in the mouth and throat, rather than travelling into the lungs. Nicotine replacement therapies are generally classified as medicinal products and are regulated under the Human Medicines Regulations in the United Kingdom.

In addition to passive nicotine delivery devices such as the inhalator, active nicotine delivery devices exist in the form of electronic cigarette which generally use heat and/or ultrasonic agitation to vaporize/aerosolise a formulation comprising nicotine and/or other flavouring, propylene glycol and/or glycerol into an aerosol, mist, or vapour for inhalation. The inhaled aerosol mist or vapour typically bears nicotine and/or flavourings without the odour and health risks associated with combustible tobacco products, and in use, the user experiences a similar satisfaction and physical sensation to those experienced from combustible tobacco products, particularly as regards exhalation because aerosol mist or vapour is of similar appearance to the smoke exhaled when smoking a conventional combustible tobacco product.

The skilled reader should appreciate that the term "smoking-substitute device" as used herein includes, but is not limited to, electronic nicotine delivery systems (ENDS), electronic cigarettes, e-cigarettes, e-cigs, vaping cigarettes, pipes, cigars, cigarillos, vaporizers and devices of a similar nature that function to produce an aerosol mist or vapour that is inhaled by a user. Some substitute devices are disposable; others are reusable, with replaceable and refillable parts. The present invention is primarily concerned with the latter, and particularly with "active" devices which require or possess a source of power in order to effect the aerosolisation.

Smoking-substitute devices typically resemble a traditional cigarette and are cylindrical in form with a mouthpiece at one end through which the user can draw the aerosol, mist or vapour for inhalation. These devices usually share several common components; a power source such as a battery, a reservoir for holding the liquid to be vaporized (often termed an e-liquid), a vaporization component such as a heater for atomizing and/or vaporizing the liquid and to thereby produce an aerosol, mist or vapour, and control circuitry operable to actuate the vaporization component responsive to an actuation signal from a switch operative by a user or configured to detect when the user draws air through the mouthpiece by inhaling.

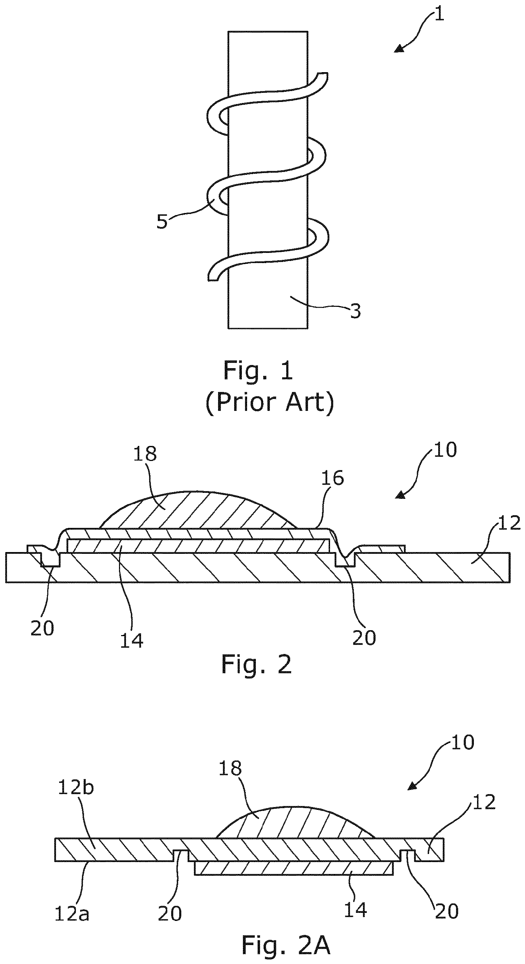

The most common form of active smoking substitute device is known as a wick-and-coil device, an example of which is schematically depicted in Figure 1. The vaporisation component comprises a wick (3), which may be solid or flexible, saturated in e- liquid with a heating coil (5) wrapped around it. The wick-and-coil arrangement is usually disposed inside a fluid-containing reservoir in order that liquid therein can be absorbed by the wick. The complete assembly is often termed a "cartomizer" (being a conflation of the words cartridge and atomizer). In use, an electric current is passed through the coil (5) resistively heating it, such heat being transferred to the e-liquid in the wick (3) causing it to evaporate. The usually soaked wick (3) generally contains more e-liquid than would be vaporised during a single inhalation. This increases the thermal mass of the wick (3) and means that the heat generated by the coil (5) is unnecessarily expended in heating all of the e-liquid rather than the amount that actually needs to be vaporised. Heating surplus liquid reduces the energy efficiency of the device. Furthermore, the coil (5) is spaced apart from the wick (3) to prevent the coil (5) from burning the wick (3). This reduces heat transfer to the wick and means that the coil (5) has to be excessively powered to compensate for the radiative dissipation of heat from the coil and inefficiencies of heating a large substrate and volume of liquid. This again reduces the energy efficiency of the device. Moreover, surplus e-liquid and repeated heating to a higher temperature increases the risk that a user will receive a larger dose of nicotine than intended and increases the potential for degradation of both nicotine and excipients.

Another problem with known e-cigarette heaters is that their design does not lend itself to automation.

A further problem with known e-cigarettes is that a user can refill their device with e-liquids which are not intended for that device and consequently may have higher levels of nicotine or additives which undergo an adverse reaction upon heating. As a result, a user may be exposed to excessive levels of nicotine or potentially harmful by-products.

WO2016/005533 discloses a substantially flat aerosol-forming cartridge for use in an electrically operated aerosol-generating system. The cartridge comprises a base layer comprising a plurality of cavities and a plurality of aerosol-forming substrates arranged on the base layer and comprising a tobacco- containing material with volatile tobacco flavour compounds which are releasable from the aerosol-forming substrate. The base layer and the plurality of aerosol-forming substrates are in contact at a contact surface which is substantially planar, and the cartridge further includes at least one electric heating element to heat the aerosol-forming substrates. Ideally, the cartridge is initially a sealed item, and prior to insertion into the body of an inhalation device, the seal is broken or removed, and after insertion, a mouthpiece component is then also attached to the inhalation device body. The mouthpiece and the cartridge are separate components.

US2014/0060554 describes an electronic smoking article comprising one or more microheaters. that provides for improved aerosol delivery. Particularly, the microheaters provide for improved control of vaporization of an aerosol precursor composition and provide for reduced power requirements to achieve consistent aerosolization. The document further relates to methods of forming an aerosol in a smoking article. The electronic smoking article can include a mouthpiece component within which the microheaters may be disposed and/or contained. In some embodiments, the microheaters are provided on or within a substrate, and the substrate may be contained within the mouthpiece. In other embodiments, the microheaters are provided within the body of the smoking article, and a replaceable substrate comprising an aerosol precursor composition is inserted into the electronic smoking article.

US2013/0255702 describes a smoking article incorporating a conductive substrate, and specifically a conductive substrate useful for Joule heating, such as in an electronic smoking article. In particular, the document describes a resistive heating element formed of a conductive substrate. The conductive substrate comprises an electrically conductive material and a carbonaceous additive, such as a binder material. The conductive substrate is carbonized in that it is subjected to calcining conditions to effectively reduce the carbonaceous additive to its carbon skeleton. It has been found that such carbonized substrate has surprisingly improved resistance properties in relation a substrate of the same formulation that is not carbonized. The carbonized substrate can include an aerosol precursor material. The formed resistive heating element can be included in an electronic smoking article to simultaneously provide resistive heating and aerosol formation with a single, unitary component. A mouthpiece component is also described which contains the conductive substrate, which forms an integral part thereof.

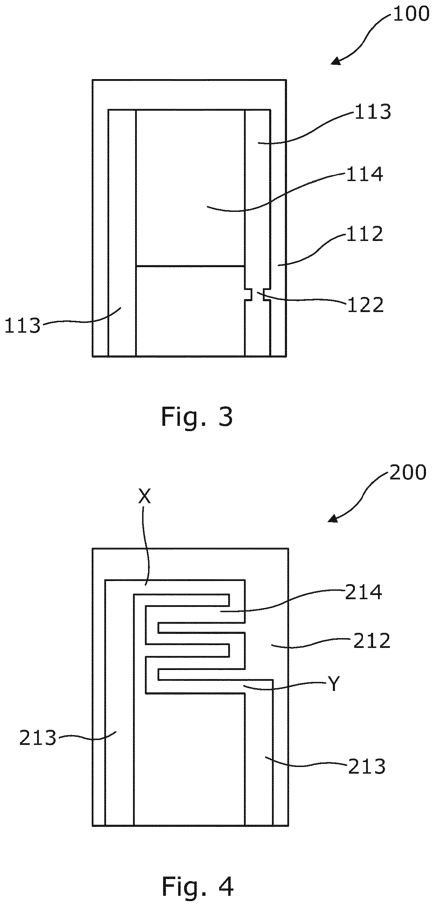

WO2016/005530, being very similar in disclosure to WO2016/005533 abovementioned, also discloses a substantially flat aerosol-forming cartridge for use in an electrically operated aerosol-generating system. Figures 3A, 3B in particular disclose a cartridge having upper and lower parts which together internally define a recess, and between which is encased both a heater and an aerosol-forming substrate which are in contact at a contact surface which is substantially planar.

The popularity and use of smoking-substitute devices has grown rapidly in the past few years. Although originally marketed as an aid to assist habitual smokers wishing to quit combustible tobacco, consumers are increasingly viewing smoking substitute devices as desirable lifestyle accessories. Furthermore the change in regulatory paradigm to that of a tobacco harm reduction one has further boosted consumer uptake of these products. This has caused concern that smoking-substitute devices may be becoming attractive to children, young adults and those currently not engaged in consumption of combustible tobacco products. Furthermore, there is on-going scientific debate about the long-terms effects on health from the prolonged use of smoking-substitute devices and concerns, particularly from healthcare professions, regarding the lack of information available to consumers regarding the use of smoking-substitute devices and associated liquids that prevent them from making informed decisions regarding their use. One area of particular concern is the quality and provenance of many e-liquids currently available on the market.

In response to safety and quality concerns, the European Union has agreed a revised Tobacco Products Directive (Tobacco and Related Products Regulations 2016). The TPD has introduced regulations applicable to smoking-substitute devices that will:
- limit the risks of inadvertent exposure to nicotine by setting maximum sizes for refill reservoirs, containers, tanks, and cartridges (Article 20.3(a))
- limit the concentration of nicotine in the liquid to 20 mg/ml (Article 20.3(b)).
- prohibit the use of certain additives in the liquid (Article 20.3(c))
- require that only high-purity ingredients are used in the manufacture of liquids (Article 20.3(d)).
- require that all ingredients (except nicotine) do not pose a risk to human health in heated or unheated form (Article 20.3(e))
- require that all smoking-substitute devices deliver doses of nicotine at consistent levels under normal conditions of use (Article 20.3(f))
   require that all products include child and tamper-proof labelling, fasteners and opening mechanisms (Article 20.3(g)).
- require that all products meet certain safety and quality standards and to ensure that products do no break or leak during use or refill (penultimate and final sentences, paragraph 41 of the recitals).

However, even despite the introduction of such nicotine dosing control measures on manufacturers and suppliers of nicotine-containing formulations intended for use in electronic cigarettes, wick-and-coil devices are inherently rudimentary and as a result will always suffer significant variability in dose between inhalations. Furthermore, because such devices require refilling by end users over which legal frameworks such as the TPD above inevitably have little or no control, such end users will always be capable of using their own, possibly adulterated liquid formulations, possibly to the detriment of their own health and that of others.

Aspects and embodiments of the invention were devised with the foregoing in mind.

### Brief Summary of the Present Invention

In a first aspect, there is provided an assembly for an inhalation device as prescribed in claim 1 hereof.

An advantage of using a heater to heat the composition compared to the medicinal inhaler or inhalator devices of the prior art described above is that the formulation can be specifically designed to deliver the compound of interest either to the lung or to the buccal cavity. For compositions containing nicotine, this means that the user experiences an enhanced "hit", i.e. an increased rate of absorption of nicotine. Consequently, this may assist in allaying a craving for nicotine more quickly, with fewer inhalations, thereby helping a user to gradually reduce their intake of nicotine.

A further advantage of the heater of the present invention compared to, for example, the heater of a conventional e-cigarette, is that the composition can be placed in direct contact with the substrate and thus be conductively heated. Heat is conductively transferred from the resistive element portion directly into the composition. In one particular embodiment in which the heater is applied to one surface of the substrate and the composition applied to another opposite surface of the substrate, but in the same general region thereof as that of the other surface over which the heater is applied, the heat from the heater is first conducted through the material of the substrate before being conductively transferred directly into the composition. In both cases, there is no space for air gap between the composition and the heater.

This means that the heater can vaporise the required amount of liquid at much lower temperatures compared to the wick-and-coil heaters of the prior art. This increases energy efficiency and reduces degradation of the heater.

When a composition is provided on the heater, the heater is configured to heat the composition so that at least a proportion of the composition vapourises or aerosolises. The skilled person should understand that an "aerosolised composition" and cognate expressions thereof appearing herein are not limited to an aerosol per se but may also comprise a proportion of the composition in the vapour phase.

Furthermore, the amount of composition deposited on the heater can be carefully controlled so that the heater only heats the necessary amount of composition. Therefore, the energy lost, for example, by heating the excess e-liquid in an e-cigarette is eliminated. As a result, the heater of the present invention has a much lower thermal mass and requires less energy to heat than the heaters of the prior art. This benefit combined with lower heated temperatures assists in increasing the efficiency of the device. Furthermore, this avoids the repeated heat-cool cycle observed in e-cigarettes which may lead to in-use instability of formulation and formation of toxicants.

In the embodiment where the composition is deposited on the same surface of the substrate to that to which the heater has been applied, the heater, or the at least one resistive element portion thereof, may further comprise a barrier layer for inhibiting undesirable by-products generated during the heating of the resistive element portions from mixing with the composition.

Depending on how they are formed, some resistive heaters release undesirable bi-products when they are heated by the application of an electric current. For example, materials or chemicals which are added to the resistive heater during manufacture are sometimes released as volatiles which may react with other chemicals during heating to form potentially harmful by-products. It is preferable that a user does not inhale these by- products. The barrier layer assists in inhibiting undesirable by-products generated during the heating of the at least one resistive heater from mixing with the composition or aerosolised composition by providing a physical barrier or obstacle between the resistive heater and the composition.

Optionally, the barrier layer may be formed of a material selected from one or more of a ceramic, a plastic and glass. These materials have been found to be suitable at providing an effective barrier layer.

In an alternative embodiment, where the composition is deposited on an opposite surface of the substrate to that to which the heater has been applied, the substrate itself provides a barrier to prevent undesirable by-products of heating from mixing with the composition.

The heater may comprise at least two contacts supported by the substrate, wherein a first end of each of the at least two contacts is connected to the resistive element portion and a second end of each of the at least two contacts is arranged to be connectable to an electric power source. This allows the second end of each of the two contacts to be connected to a power source which is separate or remote from the substrate. For example, the second ends could form part of a connector which is configured to connect to a complementary connector that in turn is connected to a power source.

The application of the resistive element portion and the contacts to one or other surface of the substrate may be achieved by a variety of different techniques, such as screen printing, thin and/or thick film printing, laser ablation, or some combination of these techniques. An advantage of printing the resistive element portion and/or contacts is that it is cost-effective and automatable, which contrasts with the slow manual process of winding a coil around a wick.

Optionally, the at least one resistive element portion and contacts may be formed of the same material but the at least one resistive element portion has a smaller cross-sectional area than the contacts such that it has a higher resistance. This allows the resistive element portion and contacts to be deposited in a single print run.

Optionally a part of the material deposited during the single print run may be ablated, for example by laser etching, to form at least one resistive element portion having a region of reduced cross-sectional area such that the region of reduced cross-sectional area has a relatively higher resistance than the remainder of the material. This step reduces the printing step to a single print run over the entire area to be occupied by the resistive element portion such that any detail or finishing required can be provided later by the ablating step.

Alternatively, the at least one resistive element portion and contacts may comprise different materials and be deposited on the substrate using separate print runs. This provides flexibility in the process and allows the properties of the resistive element portion and conductors to be modified by modifying the proportions of various material constituents contained therein.

The at least one resistive element portion may have a length longer than the straight-line distance between the points where the at least one resistive element portion is connected to the contacts. This increases the resistance of the resistive element portion. The resistance of the resistive heater can be controlled by changing the length of the resistive element portion.

Optionally, the at least one resistive element portion may follow a meandering path between the conductors. This has been found to provide a space-efficient configuration of the at least one resistive element portion.

Optionally, the at least one resistive element portion comprises one of carbon or other elements such as silver, ruthenium, palladium. Carbon has been found to have suitable resistive properties for the heater of the present invention. Silver on the other hand has a relatively high temperature coefficient of resistance compared to carbon, and the use of resistive element portions comprising silver results in a greater increase in resistance compared to the use of carbon alone. This makes it easier to monitor changes in resistance and hence the temperature of the resistive element portion.

Optionally, the heater resistive element portions may have a resistance of between 5 ohms and 15 ohms at a temperature of 130°C. This has been found to be a particularly suitable resistance for the resistive element portions and the temperature represents a relatively low operating temperature compared to, for example, a conventional e-cigarette. This resistance range also enables energy to be input to the heater using a standard lithium polymer battery whilst enabling differentiation in resistances between temperatures.

The heater may comprise a plurality of resistive element portions and a corresponding number of contacts. This provides flexibility as to which heaters are activated at any one time.

Optionally, the conductors may comprise a contact for each of the plurality of resistive element portions and a further contact which forms a common ground for each of the plurality of resistive elements portions. This provides a space-efficient arrangement on the substrate.

The substrate may be substantially rigid, and substantially planar. This assists in reducing deformation of the substrate during heating of the resistive element portion and allows a force to be applied to the substrate to help with inserting the substrate into an inhalation device.

Optionally, the substrate may comprise a material selected from one or more of a ceramic, a plastic or glass. These materials have been found to be particularly suitable for the substrate of the present invention at least in terms of their thermal and mechanical properties.

The substrate may comprise an indentation, formed for example by laser cutting, in the region surrounding the at least one resistive element portion or plurality of resistive element portions. The indentation reduces the cross-sectional area of the substrate in the region surrounding the resistive element portion thereby reducing heat transfer away from the resistive element portion through the substrate. This reduces the thermal mass (i.e. the amount of the substrate which needs to be heated during a heating cycle) of the part the substrate underlying the resistive heater, which means that less energy is required to heat this part of the substrate. Accordingly, the energy efficiency of the heater is increased. The indentation may also serve to prevent migration of formulation from the resistive heater area.

The at least one resistive element portion or at least one of the contacts may have a region of reduced cross-sectional area such that the reduced cross-sectional area region acts as a fuse which fails if the electric current flowing through the reduced cross-sectional area region exceeds a certain threshold value. The fuse acts as a safety device which prevents overheating of the heater. The fuse also acts as a failsafe in the event other safety precautions fail, for example, in the event the electronic or software control of an aerosol generation device fails. This assists in the heater complying with the strict safety regulations in place for medical devices.

The deposition of the aerosolizable composition on the substrate may occur at the time of manufacture using, for example, screen printing techniques such that the substrate is provided both with a heater and already charged with a composition to be aerosolised. The composition could comprise a predetermined number of doses. Optionally, the composition may comprise nicotine.

The attachment of the first and second mouthpiece parts may be achieved by means of snap fit connectors provided one or both of the first mouthpiece part and the second mouthpiece part.

In most preferred embodiments, the at least one resistive element portion of the heater is arranged in the vicinity of an outlet of the fluid communication means, for example an airflow channel, provided internally within the mouthpiece. This reduces the length and/or surface area of the airflow channel on which the aerosolised composition could condense, if indeed aerosolised composition has time to reach the internal surfaces of the airflow channel before exiting through the mouthpiece outlet.

The airflow channel may comprise rails for holding the heater within the interior of the airflow channel. The airflow channel may comprise first and second portions between which the heater may be disposed such that air flowing within the first portion flows over and above the surface of the heater on which the composition has been deposited, and air flowing within the second portion flows underneath the heater, beneath and over the opposite surface of the substrate to that one which the composition has been deposited.

The first and/or second airflow channel portion may be defined by at least one flat surface, which assists in creating a laminar airflow past the heater thus inhibiting the aerosolised composition from contacting the internal surfaces of the airflow channel.

The airflow channel may include a constriction orifice for restricting the flow of air therewithin. Employing a constriction orifice within the airflow channel results in a pressure drop within the airflow channel and may permit the velocity of the airflow to be controlled more accurately (e.g. by the Venturi effect) in the region of the constriction orifice allowing airflow over the heater to travel faster compared to the airflow entering the mouthpiece. The constriction orifice also restricts the flow of air through the airflow channel which provides a similar user experience to inhaling through a conventional cigarette.

Optionally, the constriction orifice may be located upstream of the heater. This provides time and space to assist the turbulent air exiting the constriction orifices in returning to laminar flow by the time it passes over the heater.

Optionally, both the first and second airflow channel portions may comprise a constriction orifice, and ideally the dimensions and fluid flow characteristics are selected such that the airflows in the first and second airflow channel portions are similar, i.e. air flowing in channel portion is travelling at generally the same speed and mass flow rate.

The mouthpiece and heater assembly may together form a replaceable consumable item which, when new, comes already charged with a composition, and which can be simply disposed of when all the composition initially present has been aerosolised and the consumable item is thus spent.

In a further aspect of the present invention, there is provided an inhalation device comprising the above-described mouthpiece and heater assembly, a main body part, the main body part comprising: a power source for the device; and a control unit.

Such an inhalation device may provide controlled and accurate dosing, would require minimal maintenance (e.g. no cleaning of the mouthpiece is necessary), and would be more hygienic (e.g. reduces build-up of residues from previous use within the inhalation device). Such an inhalation device may also maintain a more consistent level of performance (e.g. avoid blockages within the mouthpiece) since the mouthpiece can be replaced.

The main body part of such an inhalation device may additionally include may comprise a fluid inlet and fluid outlet in communication with one another, the latter of which cooperates with the fluid inlet of the mouthpiece when connected to the main body part thus completing the airflow channel.

Optionally, the main body part fluid inlet may be located proximate the end of said main body part free to which the mouthpiece is attached.

### Brief Description of the Drawings

One or more specific embodiments in accordance with aspects of the present invention will be described, by way of example only, and with reference to the following drawings in which:
Figure 1 is a schematic diagram of a prior art e-cigarette wick-and-coil heater.
Figures 2, 2A provide cross-sectional views of heaters in accordance with different embodiments of the present invention wherein an amount of a composition is deposited on, firstly, the same surface of the substrate as that over which the heater is applied, and secondly on the opposite surface as that over which the heater is applied.
Figure 3 is a schematic plan view of a heater in accordance with an embodiment of the present invention.
Figure 4 is a schematic plan view of a heater in accordance with an embodiment of the present invention.
Figures 5a - 5d are plan views of a heater in accordance with an embodiment of the present invention during various stages of manufacture.
Figure 6 is a side view of an inhalation device in accordance with an embodiment of the present invention.
Figures 7a and 7b are side views of a mouthpiece for the device of Figure 6 shown in disassembled and assembled form respectively.
Figure 8 is a cross-sectional view of the device of Figure 6 taken along the line A-A in Figure 6.
Figure 9a is a plan view of the inhalation device of Figure 6,
Figure 9b is a cross-sectional side view of the inhalation device along the line B-B in Figure 9a.
Figure 10a is a plan view of the mouthpiece according to one or more embodiments of the present invention.
Figure 10b is a cross-sectional side view of the mouthpiece along the line G-G in Figure 10a.
Figure 10c is a rear view of the mouthpiece viewed in the direction of arrow H in Figure 10a.

### Detailed Description of the Invention

Figure 2 shows a heater 10 for an inhalation device according to the present invention comprising a substrate 12 and a resistive heating element 14, which is supported by a portion of the substrate 12. The resistive element portion is connectable to an electric power source (not shown) by means of contacts (not shown). A barrier layer 16 overlies the resistive element portion 14 and part of the substrate 12. The heater 10 is shown with an amount of composition 18 which has been deposited on the barrier layer 16.

When an electric current flows through resistive element portion 14 the temperature of the resistive element portion 14 increases and heat is transferred through the barrier layer 16 to composition 18. At least a portion of composition 18 vaporises and is dispersed into the air above the heater 10. As the composition 18 evaporates away from the heater it cools and some of the vaporised composition will condensed to form liquid droplets of composition suspended in air, i.e. an aerosolised composition. This aerosolised composition can be inhaled by a user.

The barrier layer 16 provides a seal over the resistive element portion and part of the substrate, which inhibits undesirable by-products that may be generated when the resistive heater element is heated from mixing with the composition 18 or evaporating and mixing with the aerosolised composition which is inhaled by a user.

Indentations 20 have been formed in the substrate 12 near to either side of resistive heater element 14. Although not shown in the cross-section of Figure 2, it will be appreciated that indentations 20 extend in a direction into and out of the plane of the cross-section to form a trench on either side of resistive element portion 14. Further indentations (not shown) may also be formed parallel to the plane of the cross-section near to the other two sides of the resistive element portion 14. Indentations or trenches are therefore formed in the region surrounding the at least one resistive element portion 14. The indentations 20 reduce the cross-sectional area of the substrate 12 and hence heat transfer through the substrate in the region of the indentations 20. This provides a degree of thermal isolation for the region of the substrate 12 underlying the resistive element portion 14 and inhibits heat being dissipated throughout the whole of the substrate. This reduces the volume of the substrate 12 being heated by the resistive element portion 14 during any particular heating cycle, i.e. the thermal mass of the heater 10. As less heat is dissipated in the substrate 12, more heat is transferred to the composition 18, thereby improving the thermal efficiency of the heater 10.

The heater 10 can be manufactured by providing a substrate 12 and forming indentations 20 in the region where the resistive element portion 14 will be supported, for example, by using a laser cutting process. A resistive element portion 14 can then be deposited in the region surrounded by the indentations 20 using a screen printing process. This deposits a thick film of conductive ink having a suitable resistance on the substrate. If contacts (not shown) are to be provided on the substrate, these can also be deposited on the substrate 12 using a screen printing process. Screen printing provides a cost-effective and automatable method of depositing the resistive element portion and contacts. The contacts will have a higher conductivity than the resistive element portion 14. An etching process may be used to finalise the outline of the screen printed features.

Substrate 12 is made from a ceramic. However, the skilled person will appreciate that materials such as a plastic or glass or a combination of the aforementioned materials may be used. The dimensions of the substrate are 15 mm long by 10 mm wide and 0.5 mm thick, which is relatively small compared to the wick-and-coil heaters of a conventional pre- cigarette. This reduces the thermal mass of the heater 10 and helps to improve thermal efficiency. The skilled person will appreciate that the substrate can have other suitable dimensions.

The conductive ink used to form the resistive element portion 14 comprises carbon particles and silver particles. Other constituents may comprise a resin or binder and a solvent. However, the skilled person will appreciate that other mixtures can be used.

The conductive ink used to form contacts comprises conductive particles, e.g. metallic particles. However, the skilled person will appreciate that other types of particles can be used, e.g. graphite particles.

The above compositions of the conductive inks may be adapted to a particular screen printing process or to achieve a desired resistance for a particular orientation/layout of resistor shape or size.

Once the resistive heater element 14 and contacts have been screen printed on the substrate 12, the heater will generally undergo a heating process in which any volatile solvents are driven off. The heater may then undergo a sintering process at a higher temperature to sinter the conductive or resistive constituents of the conductive inks.

The barrier layer 16 is made from a layer of glass, which is thermally welded to the substrate 12 and resistive element portion 14. However, the skilled person will appreciate that barrier layer 16 can be made from any suitable material which forms an effective seal against the egress of undesirable volatile by-products such as a ceramic or a plastic or a combination of any of the aforementioned materials.

In addition, a composition to be aerosolised can also be deposited on the heater 10 during manufacture such that a heater is provided already pre-charged with a composition. Such composition can also be screen printed onto the heater 10.

By contrast, in Figure 2A (in which like reference numerals have been used to those of Figure 2 to signify like parts), a heater 10 is shown in inverted orientation with the resistive element portions of the heater now provided on a bottom, downwardly facing surface 12a of the substrate 12, i.e. a first surface of the substrate, and an amount of composition 18 has been deposited on a top or upwardly facing surface 12b of the substrate 12, i.e. an opposing second surface. In this arrangement, the substrate itself provides a barrier between the composition 18 and the resistive element portions 14 of the heater, although heat therefrom is still directly conducted through the substrate 12 into the composition to cause aerosolisation thereof and for the aerosol so created to be dispersed into the air above. Again, indentations 20 may be formed in the substrate 12 near to either side of resistive element 14.

As mentioned previously, various types of conductive ink can be used to form the resistive element portions and contacts. For example, carbon-based ink can be used to form the resistive element portions, whereas ink comprising conductive elements such as metals or graphite can be used to form the contacts. Other constituents may comprise a solvent to enable such inks to be printed. In addition, one ink can be used to print both the resistive element portions and the contacts . Ceramic and glass inks both contain a glass phase which provides the resistivity, metallic phases which provide the conductivity and high temperature coefficient of resistance. The metallic phase may comprise elements such as, for example, silver, ruthenium, palladium or other suitable metals. The above compositions of the conductive inks may be adapted to a particular screen printing process or to achieve a desired resistance for a particular orientation/layout of resistor shape or size. Once the resistive heater element portions 14 and contacts have been printed on the substrate 12, substrate and printed heater will then generally undergo a heating process to evaporate vapourise the solvents, after which a further heating process may be used to sinter the metals and melt the glass.

Figures 3 and 4 show further embodiments of heaters according to the present invention which can be made using different manufacturing processes. It should be noted that these figures show simplified schematic views. Certain features such as the indentations and the barrier layer have been omitted for clarity. However, the skilled person will appreciate that such omitted features, and other features, could be used with these described embodiments also.

Referring firstly to Figure 3, heater 100 comprises a substrate 112, a resistive element portion 114 and two contacts 113. The resistive element portion 114 and contacts 113 are formed of different materials, i.e. they have different compositions, for example the compositions described above, such that the contacts 113 are more conductive than the resistive element portion 114. Consequently, the resistive element portion 114 and contacts are deposited in separate print runs. One print run will deposit a more resistive conductive ink to form the resistive element portion 114 and another print run will deposit a more conductive ink to form the contacts 113. Either the resistive element portion 114 can be deposited first and the contacts 113 second or vice versa.

One of the contacts 113 of heater 100 has a region of reduced cross-sectional area 122 which acts as a fuse and fails if the electric current flowing through this region exceeds a certain threshold value. Producing the region of reduced cross-sectional area can be done as part of the printing process by simply printing this pattern onto the substrate, thereby negating the need to add a further component to the heater 100. Alternatively, the fuse can be formed using an ablative process such as laser cutting. The fuse acts as a safety device and prevents the heater 100 from overheating.

Referring to Figure 4, heater 200 comprises a substrate 212, a resistive element portion 214 and contacts 213. The resistive element portion 214 and contacts 213 are formed of the same material, i.e. the same conductive ink. This conductive ink will generally be more conductive than the conductive ink used to print a standalone resistive heater element or may comprise composition having a conductivity between the two compositions described above. The resistive element portion 214 is formed by providing a printed track of conductive ink having a smaller cross-sectional area or thinner width or thickness than the remainder of the printed track such that it has a higher resistance. The remainder of the printed track, i.e. the part having the larger cross-sectional area or wider width or thickness forms the contacts 213.

The resistance of the resistive element portion 214 can also be increased relative to the resistance of the contacts 213 by making the resistive heater element longer than the straight-line distance between the points X and Y where the resistive element portion 214 is connected to the contacts 213. This is achieved by giving the resistive element portion 214 a meandering or undulating pattern.

As a result of the resistive element portion 214 and contacts 213 being formed of the same material, these features can be deposited on the substrate 212 in a single print run. The pattern of the resistive element portion 214 can either be printed onto the substrate or the resistive heater element 214 can be printed as a larger block and the pattern achieved by ablating a part of the resistive heater block, for example, using a laser etching or cutting process.

In Figures 3 and 4, contacts 113 and 213 extend to and terminate at an edge of the substrates 112 and 212 respectively. This arrangement means that the heaters 100 and 200 are connectable to an electric power source (not shown) which is separate from or remote to the heater. For example, the edge of the substrates 112 and 212 could be inserted into a connector so that the contacts 113 and 213 make electrical contact with connections to an electric power source.

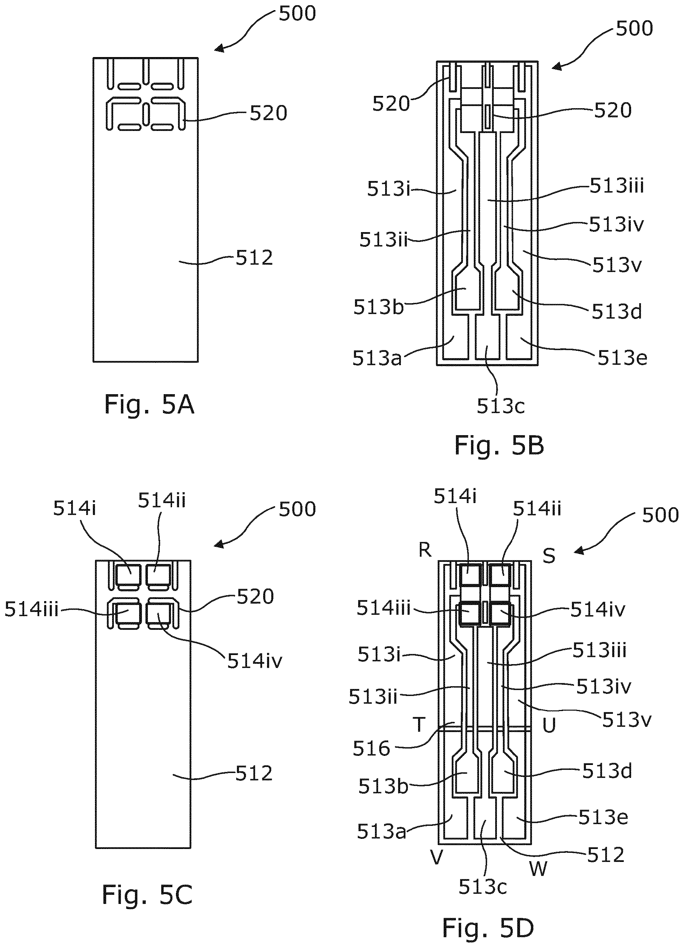

Figures 5a - 5d show a heater of the present invention during various stages of manufacture. Referring firstly to Figure 5a, a heater 500 comprises a substrate 512 having a series of indentations 520 formed in a surface of the substrate 512. The heater 500 is configured to support four resistive heater elements (not shown in Figure 5a) arranged in a 2 x 2 configuration at one end of the substrate 512. The indentations 520 are arranged in the region surrounding each of the resistive element portions. Not all the indentations 520 are joined together such that there is a gap between some of the indentations in which gap the substrate 512 has its full thickness. This is to avoid overly weakening the substrate 512 in the region of the four resistive element portions. The indentations 520 could be formed by a suitable ablative process for example laser etching or cutting.

Figure 5b shows the substrate of Figure 5a in which an arrangement of contacts 513i- 513v is supported on the substrate 512. The contacts 513i-513v have been deposited using a screen printing process. A first end of each of the contacts 513i-513v is arranged to be connected to the resistive element portions (not shown in Figure 5b) at one end of the substrate 512. Conductor 513iii is configured as a common ground connection and is arranged to be connected at its first end to each of the resistive element portions. Conductor 513iii is arranged in the middle of the contacts 513i-513v and resistive heater elements as this is the most convenient arrangement whereby it can be connected to each of the resistive element portions. Contacts 513i, 513ii, 513iv and 513v are arranged to be connected at their first ends to a respective one of each of the four resistive element portions.

A second end of the contacts 513i-513vterminates in a respective series of contact pads 513a-513e at an end of the substrate 512 opposite the end where the resistive heater elements are located. Contact pad 513c is configured to be connectable to a common ground or negative potential of an electric power source such that each of the resistive heater elements can be connected to a ground potential via conductor 513iii. Contact pads 513a, 513b, 513d and 513e are configured to be connectable to a an electric power source such that a potential difference can be generated across each of the resistive element portions via one of contacts 513i, 513ii, 513iv and 513v and common ground conductor 513iii.

Figure 5c shows substrate 512 supporting four resistive heater elements 514i - 514iv. Contacts 513i-513v have been omitted for the sake of clarity. Resistive heater elements 514i - 514iv are arranged in a 2 x 2 pattern at one end of substrate 512. Each of resistive element portions 514i - 514iv is surrounded by a formation of indentations 520. The resistive element portions 514i - 514iv have been deposited using a screen printing process.

Figure 5d shows a fully assembled heater 500 comprising substrate 512, contacts 513i- 513v, resistive element portions 514i - 514iv, barrier layer 516 and a composition containing nicotine (not shown) deposited on each of the resistive element portions 514i - 514iv. Each of the resistive element portions 514i - 514iv has been connected across a respective one of the contacts 513i, 513ii, 513iv and 513v and the common ground conductor 513iii. When a potential difference is generated across one of the resistive heater elements 514i - 514iv an electric current flows through the resistive element portion, thereby activating the resistive element portion and causing its temperature to increase. For example, applying a positive potential to contact pad 513a and a ground or negative potential to contact pad 513c activates resistive heater element 514i and causes it to generate heat. Each of the resistive element portions 514i - 514iv is therefore independently activatable by applying a positive potential to any one of contact pads 513a, 513b, 513d and 513e and a ground potential to contact pad 513c.

The barrier layer 516 provides a seal over the resistive element portions 514i - 514iv and part of the contacts 513i-513v. The barrier layer 516 extends over an area of the heater 500 denoted by points RSTU in Figure 5d. The area of the heater denoted by points TUVW is not covered by the barrier layer so not to insulate the contact pads 513a-513e and allow these to make an electrical connection to an electric power source.

The nicotine containing compositions (not shown) are deposited on top of the barrier layer 516 above each of the resistive element portions 514i - 514iv. The compositions contain 0.5mg of nicotine in total (at 40% concentration). A screen printing process has been used to deposit the compositions, although the skilled person will appreciate that other methods of deposition could be used. The amount of nicotine containing composition deposited above each of the resistive element portions 514i - 514iv may comprise a single or multiple doses of nicotine per inhalation.

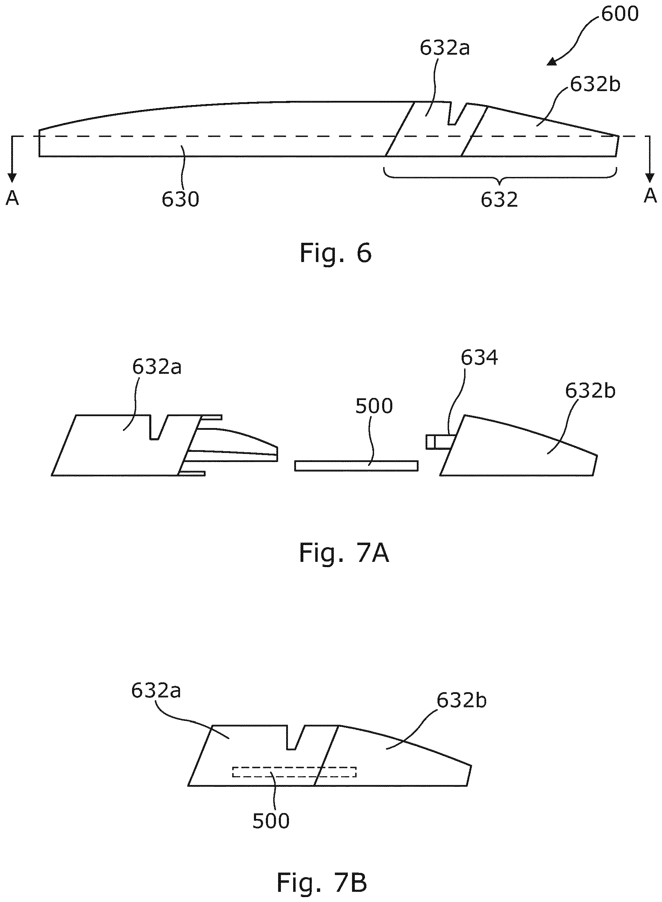

Figure 6 shows an inhalation device 600 according to the present invention comprising a main body part 630 and a mouthpiece 632. The mouthpiece 632 is releasably attachable to the main body part 630. Furthermore, the mouthpiece 632 is formed of separate first 632a and second 632b parts which are assembled during manufacture. However, the skilled person will appreciate that the inhalation device 600 can also be formed from a single piece, e.g. a single tube.

Figure 7a shows the mouthpiece 632 in disassembled form. First mouthpiece part 632a has a slot (not shown) for receiving the heater 500 of Figure 5d. During manufacture, heater 500 is inserted into the slot or recess of first mouthpiece part 632a and is held in place by the attachment of the second mouthpiece part 632b to the first mouthpiece part 632a. The second mouthpiece part 632b is attached to the first mouthpiece part 632a by means of snap fit connectors 634 on either side of the second mouthpiece part 632b.

Figure 7b shows the mouthpiece 632 in assembled form. Heater 500 is held securely within the mouthpiece 632. As described above, heater 500 comprises nicotine containing compositions deposited on the resistive element portions and therefore the mouthpiece 62 comprises a replaceable consumable which can be releasably connected to the main body part 630 of inhalation device 600.

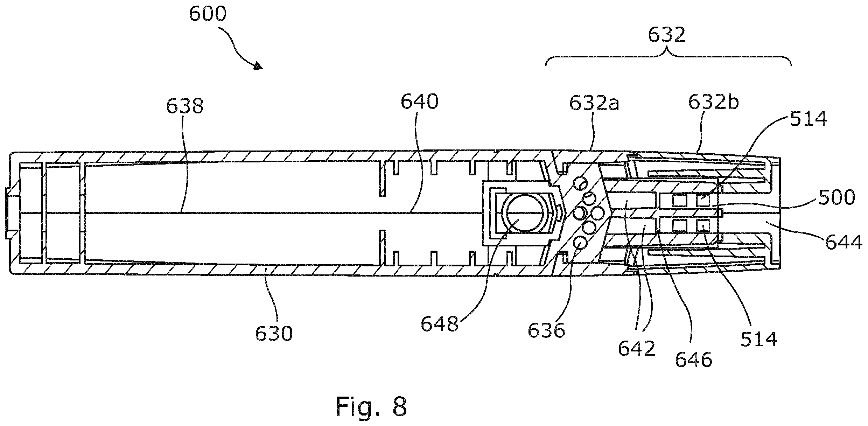

Figure 8 shows a cross-section through inhalation device 600 along the line A-A in Figure 6. Mouthpiece 632 containing heater 500 is connected to main body part 630. The end of main body part 630 to which mouthpiece 632 is connected comprises a number of contact pins 636 which are arranged to make electrical contact with respective ones of contact pads 513a-513e of heater 500.

The main body part has a first interior space 638 for accommodating an electric power source (not shown) and a second interior space for containing a control unit (not shown) for controlling electrical activation of resistive element portions 514. Contact pins 636 are connected to the electric power source via the control unit. A button 648 is also provided on the main body part 630 to enable a user to activate the heater 500. Alternatively, the skilled person will appreciate that a sensor responsive to a user's inhalation could be used to activate the heater.

Mouthpiece 632 has channels 642 which overlie resistive element portions 514 when heater 500 is installed in the mouthpiece 632. The channels 642 are in fluid communication with an air inlet (not shown) arranged on the main body part 630 and an air outlet 644 of the mouthpiece 632. A constriction 646 is arranged in channels 642 immediately prior to the resistive element portions 514 to accelerate the airflow and provide a pressure drop in this region of the channel. This assists entrainment of the aerosolised composition in the airflow.

The device 600 of Figures 6 to 8 is configured to be highly accurate and to comply with the requirements of the Human Medicines Regulations Such a device is therefore suitable as a nicotine replacement therapy.

In use, a user will seal his lips around the mouthpiece 632 of inhalation device 600 and inhale. Air is drawn into the air inlet, through channels 642 and over the heater 500 in the region of resistive element portions 514 before exiting the inhalation device via air outlet 644. At the same time as inhaling the user presses button 648 to activate heater 500. Dependent on the dose to be delivered, the control unit will activate one or more of resistive element portions 514 by directing an electric current through these resistive element portions 514 causing them to generate heat. At least a portion of the compositions deposited above the respective one or more resistive element portions is vaporised and forms an aerosolised composition above heater 500 which becomes entrained in the moving airflow. Since the composition is in direct conductive contact with the heater, aerosolisation of the required amount of composition can be achieved at much lower temperatures, i.e. 140°C, compared to conventional wick-and-coil heaters which typically heat to around 300°C. The aerosolised composition is then inhaled by a user via outlet 644. The device then resets in preparation for the next inhalation.

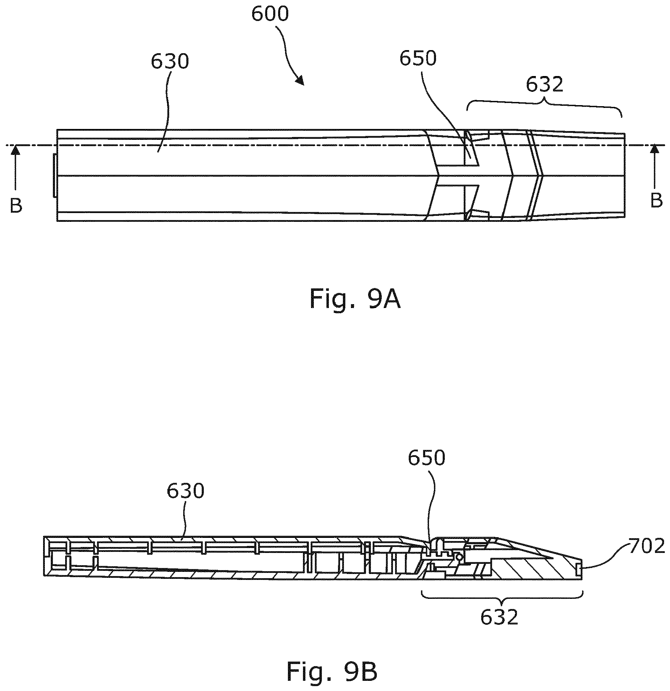

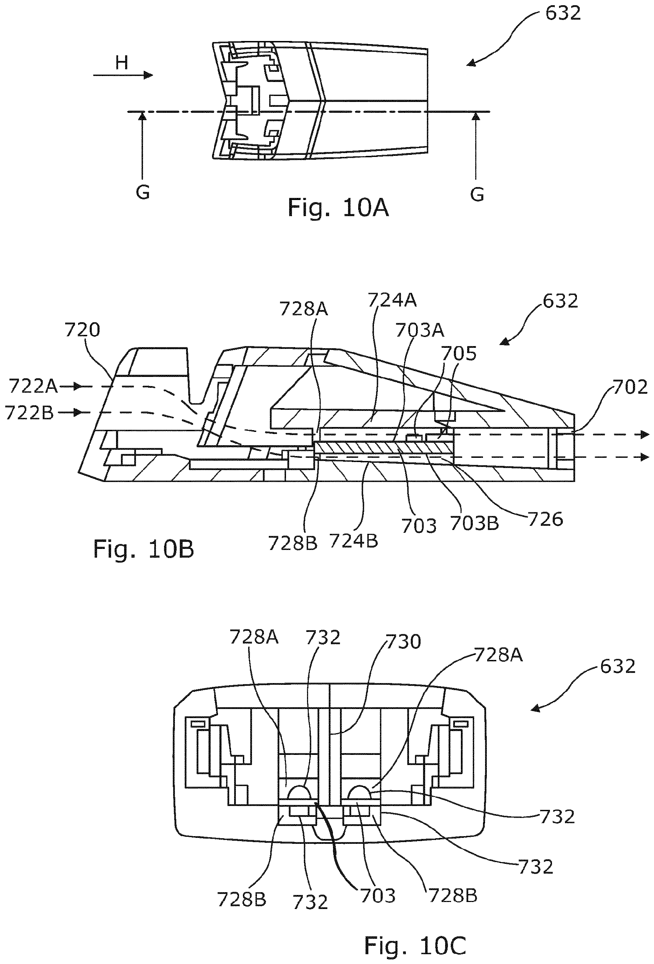

Referring now to Figures 9a and 9b, the inhalation device 600 is shown with air inlet 650 arranged in a top surface of the main body part 630. The air inlet 650 is laterally spaced apart from the central longitudinal axis of the inhalation device 600 and is located in the region where the mouthpiece 632 attaches to the main body part 630. Figure 9b shows a cross-sectional view through inhalation device 900 along the line B-B in Figure 9a. The air inlet 650 is in fluid communication with the mouthpiece 632 and air exits the inhalation device 600 via an outlet 702 (part of which is shown in Figure 9b). An airflow channel passes from the air inlet 650 on the main body part 630 to the outlet 702 of the mouthpiece 632. The main portion of the airflow channel which passes through the mouthpiece 632 is not visible in Figure 9b because it passes closer to the central longitudinal axis of the device, i.e. in the region of line G-G in Figure 10a.

Referring to Figure 10a, this shows a plan view of the mouthpiece 632 alone, i.e. detached from the main body part 630. Figure 9b shows a cross-sectional view through the mouthpiece along the line G-G in Figure 10a. Air enters the mouthpiece 632 via an opening 720 at the rear of the mouthpiece 632, which opening 720 is in fluid communication with the air inlet 650 (see Figures 9a and 9b). The air flows through the mouthpiece 632 to the outlet 702 via an enclosed airflow channel or fluid passage. The airflow through the airflow channel is denoted by dotted lines 722a and 722b in Figure 10b.

A heater 703 is arranged inside the mouthpiece 632 within the airflow channel. In the vicinity of the heater 703, the airflow channel comprises a first airflow channel portion 724a and a second airflow channel portion 724b. The first airflow channel portion 724a is arranged to direct a portion of the airflow (denoted by dotted line 722a) past and above the first upwardly facing surface 703a of the heater 703 and its the resistive element portions 705. The resistive element portions 705 are located at the downstream end of the heater 703 in the vicinity of or near to the outlet 702 of mouthpiece 632. The second airflow channel portion 724b is arranged to direct a portion of the airflow (denoted by dotted line 722b) past and beneath the second downwardly facing surface 703b of the heater 703. The upper and lower surfaces of the first 724a and second 724b airflow channel portions respectively are flat to encourage laminar airflow past the resistive element portions 705.

The heater 703 is supported on rails 726 which run parallel to the longitudinal axis of the mouthpiece and hold the heater at a central region within the airflow channel such that air can flow both above and below the heater 703. Protrusions 728a and 728b extend from the upper and lower surfaces of the first 724a and second 724b airflow channel portions respectively and contact the heater 703 near its upstream end to assist in holding the heater 703 in place within the mouthpiece 632. Each of protrusions 728a and 728b has a constriction orifice or channel restriction (not shown in Figure 10b, see Figure 10c) passing through it. The purpose of the constriction orifices is to increase resistance to inhalation by restricting the airflow in the region of the protrusions 728a and 728b and provide a more realistic feel to the inhalation device 600 for smokers of traditional tobacco products. The protrusions 728a and 728b are located sufficiently upstream of the resistive heater elements 705 such that turbulent air exiting the constriction orifices has space to return to laminar flow by the time it passes over the resistive heater elements 705. Laminar flow assists in inhibiting the aerosolised composition from reaching the surfaces of the airflow channel because the aerosolised composition tends to flow through the device entrained with the streamlined flow.

Figure 10c shows a rear view of the mouthpiece 632, i.e. a view in the direction of arrow H in Figure 10a. The mouthpiece 632 has a central vertical dividing wall 730 which divides the airflow channel in two. The portion of the mouthpiece 632 to the left of the dividing wall 730 is essentially a mirror image of the portion of the mouthpiece 632 to the right of the dividing wall 730. The left-hand portion of the mouthpiece 632 repeats the features of the mouthpiece 632 to the right of the dividing wall 730.

As can be seen from Figure 10c, protrusions 728a and 728b contact the heater 703 to assist in holding it in place within the mouthpiece 632. Each of protrusions 728a and 728b has a constriction orifice 732 passing through it. The constriction orifice is semi-circular in shape, although any suitable shape can be used. The size or diameter of the constriction orifices 732 is less than the size of the airflow channel in which they are situated in order to restrict the airflow in the region of the protrusions 728a and 728b as described above.

In use, a user places the mouthpiece 632 in their mouth and inhales through the inhalation device 600. Air flows in through the air inlet 650 and through the airflow channel to the outlet 650 of the mouthpiece 632. A sensor (not shown) may be provided to detect a drop in pressure within the airflow channel and sends a signal to the control circuitry to heat or activate the resistive heater elements 705. However, the skilled person will appreciate that a button (e.g. 648, Fig. 8) pressed by the user could be used instead of a sensor to activate the resistive heater elements 705. Once activated, heat from the resistive heater elements 705 is transferred to a composition overlying the resistive heater elements 705. At least a portion of the composition evaporates to form an aerosolised composition which becomes entrained in the airflow passing over the upper first surface 703a of the heater 703 and is inhaled by the user.

Since the resistive heater elements 705 are located at the downstream end of the heater 703 in the vicinity or near to the outlet 702, there is insufficient time and/or insufficient length or surface area of the airflow channel for condensation to form. Consequently, a greater proportion of the nicotine containing composition reaches the user. Furthermore, this arrangement inhibits the formation of condensation droplets within the mouthpiece 632, which can be unpleasant if inhaled by a user.

In the described embodiment, airflow not only passes over the upper surface of the heater 703 but a portion of the airflow channel, i.e. the second airflow channel portion 724b, is located below the heater 703. It has been found by the inventors that the lower second airflow channel portion 724b may assist in inhibiting condensation of the aerosolised composition on the lower surfaces of the substrate and mouthpiece.

As the user inhales, air has to be drawn through the constriction orifices 732. As discussed above, this increases resistance to inhalation by restricting the airflow and provides a more realistic feel to the inhalation device 100 for smokers of traditional tobacco products. A constriction orifice 732 is located in both the upper first airflow channel portion 724a and the lower second airflow channel portion 724b so that the upper and lower airflows are restricted equally, i.e. both airflows are travelling at generally the same speed and mass flow rate. This assists in the smooth flow of air through the device, which further inhibits the formation of condensation.

Various modifications will be apparent to those skilled in the art. For example, the resistive element portions, contacts and compositions could be deposited by a process other than screen printing, for example, by inkjet printing or 3D printing. Additionally pellets comprising a composition could be supported by or attached to the heater. Upon the application of heat the pellets melt and release a composition which is aerosolised.

## Claims

1. An assembly for an inhalation device (630) comprising a mouthpiece (632) and a heater (500), said heater comprising a substrate (512) which supports
- at least one resistive element portion (514i, ii, iii, iv) applied over a first region of at least one of surface of said substrate,
- at least a pair of contacts (513a, b, c, d, e, 513i, ii, iii, iv, v) each connected to the at least one resistive element portion at one end of said contacts and applied over a second region of the said at least one surface of said substrate,
- said substrate first region being more proximate a forward or leading edge of said substrate, and said second region being more proximate a rearward or trailing edge thereof,
- an amount of an aerosolizable composition (18) deposited on the substrate above said first region on said at least one surface thereof or a surface opposite thereto such that heat generated by said at least one resistive element portion is directly or indirectly conducted to the aerosolizable composition to cause at least some aerosolization thereof,
said mouthpiece being provided with a fluid inlet (720) at an upstream, rear end thereof, and a fluid outlet (702) at a downstream, front end thereof, fluid communication means (724A, 724B) being provided internally of said mouthpiece between said inlet and said outlet,
said heater being disposed substantially within the mouthpiece in or adjacent said fluid communication means and arranged such that the substrate leading edge more is proximate the mouthpiece fluid outlet and its trailing edge is substantially adjacent the rear end of the mouthpiece such that at least portions of the contacts are both exposed and accessible towards the rear end of the mouthpiece, and such that when fluid is flowing through the fluid communication means and aerozolisation is simultaneously occurring, aerosol generated is entrained in the fluid flowing within said mouthpiece through said fluid communication means,
**characterised in that**
the mouthpiece comprises first and second parts (632a, 632b), said mouthpiece first part having a slot which receives the heater which is held in place within the mouthpiece when the second mouthpiece part is attached to said first mouthpiece part, the entire assembly being releasably attachable to a main body part (630) of the inhalation device at the rear end of the mouthpiece, an electrical connection with said exposed accessible contacts being achieved when said mouthpiece rear end is attached to said main body part.

2. An assembly according to claim 1 wherein said substrate supports a plurality of resistive element portions (514i, ii, iii, iv) and a corresponding number of pairs of contacts (513a, b, c, d, e, 513i, ii, iii, iv, v) connected thereto.

3. An assembly according to claim 2 wherein the heater (500) is supported within the mouthpiece (632) by means of rails (726) which run parallel to the longitudinal axis of the mouthpiece and hold the heater at a central region within the fluid communication (724A, 724B) means such that air can flow both above and below said heater.

4. An assembly according to claim 2 or 3 wherein the mouthpiece (632) is provided with a central vertical dividing wall (730) which vertically divides the fluid communication means (724A, 724B) provided internally within said mouthpiece into two separate airflow channels.

5. An assembly according to any preceding claim wherein the aerosolizable composition (18) is deposited on the same surface of the substrate (512) as that to which the at least one resistive element portion (514i, ii, iii, iv) of the heater (500) has been applied.

6. An assembly according to any of claims 1-4 wherein the aerosolizable composition (18) is deposited on the surface of the substrate (512) opposite to that to which the at least one resistive element portion (514i, ii, iii, iv) of the heater (500) has been applied.

7. An assembly according to claim 5 wherein the at least one resistive element portion (514i, ii, iii, iv) of the heater is covered by a barrier layer (16).

8. An assembly according to claim 7 wherein the barrier layer (16) is formed of a material selected from one or more of: a ceramic, a plastic and glass.

9. An assembly according to any preceding claim wherein the at least one resistive element portion (514i, ii, iii, iv) and respective contacts (513a, b, c, d, e, 513i, ii, iii, iv, v) connected thereto are formed of the same material but the at least one resistive element portion has a smaller cross-sectional areas than said contacts.

10. An assembly according to any of claims 1-8 wherein the materials of which the at least one resistive element portions (514i, ii, iii, iv) and the respective contacts (513a, b, c, d, e, 513i, ii, iii, iv, v) are constituted are different.

11. An assembly according to any preceding claim wherein the at least one resistive element portion (514i, ii, iii, iv) follows meandering paths between the points where each is connected to a respective contact (513a, b, c, d, e, 513i, ii, iii, iv, v).

12. An assembly according to any preceding claim wherein the at least one resistive element portion (514i, ii, iii, iv) comprises at least one of: carbon, silver, ruthenium, palladium.

13. An assembly according to any preceding claim wherein the at least one resistive element portion (514i, ii, iii, iv) has a resistance of between 5 ohms and 15 ohms at a temperature of 130°C.

14. An assembly according to claim 2 and any claim dependent thereon wherein the heater (500) comprises a first contact (513a, b, d, e, 513i, ii, iv, v) for each of the plurality of resistive element portions (514i, ii, iii, iv) and one further contact (513c, iii) which forms a common ground for each of the plurality of resistive elements portions and which acts as the alternate common contact in the pair of contacts between which each resistive element portion is thus connected.

15. An assembly according to any preceding claim wherein substrate (512) is one or more of: substantially rigid, substantially planar, and formed of a material which is one of: glass, ceramic, plastic.

16. An assembly according to any preceding claim wherein the substrate (512) comprises one or more indentations (520) which at least partially surround the resistive element portions.

17. An inhalation device including the assembly of any of the preceding claims and further comprising a main body part (630) which includes a power source for the device and a control unit.

## Patentansprüche

1. Anordnung für eine Inhalationsvorrichtung (630), umfassend ein Mundstück (632) und ein Heizelement (500), das Heizelement umfassend ein Substrat (512), das mindestens einen Widerstandselementabschnitt (514i, ii, iii, iv) trägt, der über einen ersten Bereich mindestens einer Oberfläche des Substrats aufgebracht ist,
- mindestens ein Paar von Kontakten (513a, b, c, d, e, 513i, ii, iii, iv, v), die jeweils mit dem mindestens einen Widerstandselementabschnitt an einem Ende der Kontakte verbunden sind und über einen zweiten Bereich der mindestens einen Oberfläche des Substrats aufgebracht sind,
- wobei der erste Bereich des Substrats mehr in der Nähe einer vorderen oder Vorderkante des Substrats ist und der zweite Bereich mehr in der Nähe einer hinteren oder Hinterkante davon ist,
- wobei eine Menge einer aerosolierbaren Zusammensetzung (18), die oberhalb des ersten Bereichs auf der mindestens einen Oberfläche davon oder einer dazu gegenüberliegenden Oberfläche auf dem Substrat abgelagert ist, sodass Wärme, die durch den mindestens einen Widerstandselementabschnitt erzeugt wird, direkt oder indirekt zu der aerosolierbaren Zusammensetzung geleitet wird, um mindestens eine gewisse Aerosolisierung davon zu bewirken,
wobei das Mundstück mit einem Fluideinlass (720) an einem stromaufwärtigen hinteren Ende davon und einem Fluidauslass (702) an einem stromabwärtigen vorderen Ende davon versehen ist, wobei Fluidkommunikationsmittel (724A, 724B) im Inneren des Mundstücks zwischen dem Einlass und dem Auslass bereitgestellt sind,
wobei das Heizelement im Wesentlichen innerhalb des Mundstücks in der oder angrenzend an die Fluidverbindungseinrichtung angeordnet ist und angeordnet ist, sodass die vordere Kante des Substrats in der Nähe des Fluidauslasses des Mundstücks ist und seine hintere Kante im Wesentlichen an das hintere Ende des Mundstücks angrenzt, sodass zumindest Teile der Kontakte sowohl freiliegen als auch in Richtung des hinteren Endes des Mundstücks zugänglich sind, und sodass, wenn Fluid durch das Fluidkommunikationsmittel strömt und gleichzeitig Aerosolisierung erfolgt, erzeugtes Aerosol in dem Fluid mitgeführt wird, das in dem Mundstück durch die Fluidkommunikationsmittel strömt,
**dadurch gekennzeichnet, dass**
das Mundstück ein erstes und ein zweites Teil (632a, 632b) umfasst, wobei das erste Teil des Mundstücks einen Schlitz aufweist, der die Heizvorrichtung aufnimmt, die innerhalb des Mundstücks an ihrem Platz gehalten wird, wenn das zweite Teil des Mundstücks an dem ersten Teil des Mundstücks angebracht ist, wobei die gesamte Anordnung lösbar an einem Hauptkörperteil (630) der Inhalationsvorrichtung an dem hinteren Ende des Mundstücks angebracht werden kann, wobei eine elektrische Verbindung mit den freiliegenden zugänglichen Kontakten erreicht wird, wenn das hintere Ende des Mundstücks an dem Hauptkörperteil angebracht ist.

2. Anordnung nach Anspruch 1, wobei das Substrat eine Vielzahl von Widerstandselementabschnitten (514i, ii, iii, iv) und eine entsprechende Anzahl von Kontaktpaaren (513a, b, c, d, e, 513i, ii, iii, iv, v) trägt, die damit verbunden sind.

3. Anordnung nach Anspruch 2, wobei das Heizelement (500) mittels Schienen (726) innerhalb des Mundstücks (632) gehalten wird, die parallel zu der Längsachse des Mundstücks verlaufen und das Heizelement in einem zentralen Bereich innerhalb des Fluidkommunikationsmittels (724A, 724B) halten, sodass Luft sowohl über als auch unter dem Heizelement strömen kann.

4. Anordnung nach Anspruch 2 oder 3, wobei das Mundstück (632) mit einer mittleren vertikalen Trennwand (730) versehen ist, die das im Inneren des Mundstücks bereitgestellte Fluidkommunikationsmittel (724A, 724B) vertikal in zwei getrennte Luftstromkanäle unterteilt.

5. Anordnung nach einem der vorherigen Ansprüche, wobei die aerosolierbare Zusammensetzung (18) auf der gleichen Oberfläche des Substrats (512) abgelagert ist wie jene, auf die der mindestens eine Widerstandselementabschnitt (514i, ii, iii, iv) des Heizelements (500) aufgebracht wurde.

6. Anordnung nach einem der Ansprüche 1 bis 4, wobei die aerosolierbare Zusammensetzung (18) auf der Oberfläche des Substrats (512) gegenüber jener abgelagert wird, auf die der mindestens eine Widerstandselementabschnitt (514i, ii, iii, iv) des Heizelements (500) aufgebracht wurde.

7. Anordnung nach Anspruch 5, wobei der mindestens eine Widerstandselementabschnitt (514i, ii, iii, iv) des Heizelements von einer Sperrschicht (16) abgedeckt ist.

8. Anordnung nach Anspruch 7, wobei die Sperrschicht (16) aus einem Material gebildet ist, das aus einem oder mehreren der Folgenden ausgewählt ist: einer Keramik, einem Kunststoff und Glas.

9. Anordnung nach einem der vorherigen Ansprüche, wobei der mindestens eine Widerstandselementabschnitt (514i, ii, iii, iv) und die jeweiligen damit verbundenen Kontakte (513a, b, c, d, e, 513i, ii, iii, iv, v) aus demselben Material gebildet sind, der mindestens eine Widerstandselementabschnitt jedoch eine kleinere Querschnittsfläche als die Kontakte aufweist.

10. Anordnung nach einem der Ansprüche 1-8, wobei die Materialien, aus denen die mindestens einen Widerstandselementabschnitte (514i, ii, iii, iv) und die jeweiligen Kontakte (513a, b, c, d, e, 513i, ii, iii, iv, v) bestehen, verschieden sind.

11. Anordnung nach einem der vorherigen Ansprüche, wobei der mindestens eine Widerstandselementabschnitt (514i, ii, iii, iv) mäanderförmigen Pfaden zwischen den Punkten folgt, an denen jeder mit einem entsprechenden Kontakt (513a, b, c, d, e, 513i, ii, iii, iv, v) verbunden ist.

12. Anordnung nach einem der vorherigen Ansprüche, wobei der mindestens eine Widerstandselementabschnitt (514i, ii, iii, iv) mindestens eines von Folgenden umfasst: Kohlenstoff, Silber, Ruthenium, Palladium.

13. Anordnung nach einem der vorherigen Ansprüche, wobei der mindestens eine Widerstandselementabschnitt (514i, ii, iii, iv) einen Widerstand zwischen 5 Ohm und 15 Ohm bei einer Temperatur von 130 °C aufweist.

14. Anordnung nach Anspruch 2 und jedem davon abhängigen Anspruch, wobei das Heizelement (500) einen ersten Kontakt (513a, b, d, e, 513i, ii, iv, v) für jeden der Vielzahl von Widerstandselementabschnitten (514i, ii, iii, iv) und einen weiteren Kontakt (513c, iii) umfasst, der eine gemeinsame Masse für jeden der Vielzahl von Widerstandselementabschnitten bildet und der als der abwechselnde gemeinsame Kontakt in dem Paar von Kontakten wirkt, zwischen denen jeder Widerstandselementabschnitt somit verbunden ist.

15. Anordnung nach einem der vorherigen Ansprüche, wobei das Substrat (512) eines oder mehrere von Folgenden ist: im Wesentlichen starr, im Wesentlichen planar und aus einem Material gebildet, das eines von Folgenden ist: Glas, Keramik, Kunststoff.

16. Anordnung nach einem der vorherigen Ansprüche, wobei das Substrat (512) eine oder mehrere Vertiefungen (520) aufweist, die die Widerstandselementabschnitte zumindest teilweise umgeben.

17. Inhalationsvorrichtung, beinhaltend die Anordnung nach einem der vorherigen Ansprüche und ferner umfassend ein Hauptkörperteil (630), das eine Energiequelle für das Gerät und eine Steuereinheit beinhaltet.

## Revendications

1. Ensemble destiné à un dispositif d'inhalation (630) comprenant un embout buccal (632) et un élément chauffant (500), ledit élément chauffant comprenant un substrat (512) qui supporte
- au moins une partie d'élément résistif (514i, ii, iii, iv) appliquée sur une première région de ladite au moins une surface dudit substrat,
- au moins une paire de contacts (513a, b, c, d, e, 513i, ii, iii, iv, v) chacun connecté à ladite au moins une partie d'élément résistif au niveau d'une extrémité desdits contacts et appliquée sur une seconde région de ladite au moins une surface dudit substrat,
- ladite première région du substrat étant plus proche d'un bord avant ou d'attaque dudit substrat et ladite seconde région étant plus proche d'un bord arrière ou de fuite de celui-ci,
- une quantité d'une composition transformable en aérosol (18) déposée sur le substrat au-dessus de ladite première région sur ladite au moins une surface de celui-ci ou sur une surface en regard de celui-ci, de sorte que la chaleur générée par ladite au moins une partie d'élément résistif soit directement ou indirectement dirigée vers la composition transformable en aérosol pour amener au moins une certaine transformation en aérosol de celle-ci,
ledit embout buccal étant pourvu d'une entrée de fluide (720) à une extrémité amont, en avant et en arrière de celui-ci, et d'une sortie de fluide (702) à une extrémité aval, en avant de celui-ci, le moyen de communication fluidique (724A, 724B) étant fourni à l'intérieur dudit embout buccal entre ladite entrée et ladite sortie,
ledit élément chauffant étant pratiquement disposé à l'intérieur de l'embout buccal ou dans ledit moyen de communication fluidique adjacent et disposé de sorte que le bord d'attaque du substrat est plus proche de la sortie de fluide de l'embout buccal et que son bord de fuite est pratiquement adjacent à l'extrémité arrière de l'embout buccal de sorte qu'au moins lesdites parties des contacts sont à la fois exposées et accessibles vers l'extrémité arrière de l'embout buccal, de sorte que lorsque le fluide s'écoule à travers le moyen de communication fluidique et que la formation de l'aérosol se produit simultanément, l'aérosol généré est entraîné dans le fluide circulant dans ledit embout buccal à travers ledit moyen de communication fluidique, **caractérisé en ce que**
l'embout buccal comprend les première et seconde pièces (632a, 632b), ladite première pièce de l'embout buccal présentant une encoche qui reçoit l'élément chauffant qui est maintenu en place à l'intérieur de l'embout buccal lorsque la seconde pièce de l'embout buccal est fixée à ladite première pièce de l'embout buccal, l'ensemble pouvant être fixé de manière amovible à une pièce du corps principal (630) du dispositif d'inhalation à l'extrémité arrière de l'embout buccal, une connexion électrique avec lesdits contacts accessibles exposés est réalisée lorsque ladite extrémité arrière de l'embout buccal est fixée à ladite pièce du corps principal.

2. Ensemble selon la revendication 1, dans lequel ledit substrat supporte une pluralité de parties d'élément résistif (514i, ii, iii, iv) et un nombre correspondant de paires de contacts (513a, b, c, d, e, 513i, ii, iii, iv, v) qui y sont connectés.

3. Ensemble selon la revendication 2, dans lequel l'élément chauffant (500) est supporté à l'intérieur de l'embout buccal (632) au moyen des rails (726) qui courent parallèlement à l'axe longitudinal de l'embout buccal et maintiennent l'élément chauffant au niveau d'une région centrale à l'intérieur du moyen de communication fluidique (724A, 724B) de sorte que l'air peut s'écouler au-dessus et en dessous dudit élément chauffant.

4. Ensemble selon la revendication 2 ou 3, dans lequel l'embout buccal (632) est pourvu d'une paroi centrale de division verticale (730) qui divise verticalement le moyen de communication fluidique (724A, 724B) fourni à l'intérieur dudit embout buccal en deux canaux d'écoulement d'air distincts.

5. Ensemble selon une quelconque revendication précédente dans lequel la composition transformable en aérosol (18) est déposée sur la même surface du substrat (512) que celle à laquelle a été appliquée ladite au moins une partie d'élément résistif (514i, ii, iii, iv) de l'élément chauffant (500).

6. Ensemble selon l'une quelconque des revendications 1 à 4, dans lequel la composition transformable en aérosol (18) est déposée sur la surface du substrat (512) en regard de celle à laquelle a été appliquée ladite au moins une partie d'élément résistif (514i, ii, iii, iv) de l'élément chauffant (500).

7. Ensemble selon la revendication 5, dans lequel ladite au moins une partie d'élément résistif (514i, ii, iii, iv) de l'élément chauffant est recouverte d'une couche barrière (16).

8. Ensemble selon la revendication 7, dans lequel la couche barrière (16) est constituée d'un matériau sélectionné parmi un ou plusieurs éléments suivants : une céramique, un plastique et un verre.

9. Ensemble selon une quelconque revendication précédente, dans lequel ladite au moins une partie d'élément résistif (514i, ii, iii, iv) et des contacts respectifs (513a, b, c, d, e, 513i, ii, iii, iv, v) qui y sont connectés sont constitués du même matériau, mais ladite au moins une partie d'élément résistif présente une superficie de section transversale plus petite que lesdits contacts.

10. Ensemble selon l'une quelconque des revendications 1 à 8, dans lequel les matériaux dont lesdites parties d'élément résistif sont constituées (514i, ii, iii, iv) et dont les contacts respectifs (513a, b, c, d, e, 513i, ii, iii, iv, v) sont constitués sont différents.

11. Ensemble selon une quelconque revendication précédente, dans lequel ladite au moins une partie d'élément résistif (514i, ii, iii, iv) suit des trajets de mesure entre les points où chacun est connecté à un contact respectif (513a, b, c, d, e, 513i, ii, iii, iv, v).

12. Ensemble selon l'une quelconque des revendications précédentes, dans lequel ladite au moins une partie d'élément résistif (514i, ii, iii, iv) comprend au moins un parmi les éléments suivants : carbone, argent, ruthénium, palladium.

13. Ensemble selon l'une quelconque des revendications précédentes, dans lequel ladite au moins une partie d'élément résistif (514i, ii, iii, iv) présente une résistance comprise entre 5 ohms et 15 ohms à une température de 130 °C.

14. Ensemble selon la revendication 2 et une quelconque revendication qui en dépend, dans lequel l'élément chauffant (500) comprend un premier contact (513a, b, d, e, 513i, ii, iv, v) pour chacun partie de la pluralité de parties d'élément résistif (514i, ii, iii, iv) et un autre contact (513c, iii) qui constitue une masse commune pour chaque partie de la pluralité de parties d'élément résistif et qui agit comme le contact commun alternatif dans la paire de contacts entre lesquels chaque partie d'élément résistif est ainsi connectée.

15. Ensemble selon l'une quelconque des revendications précédentes, dans lequel le substrat (512) est un ou plusieurs parmi les éléments suivants : pratiquement rigide, pratiquement plan et constitué d'un matériau qui est un parmi les éléments suivants : verre, céramique, plastique.

16. Ensemble selon l'une quelconque des revendications précédentes, dans lequel le substrat (512) comprend une ou plusieurs indentations (520) qui entourent au moins partiellement les parties d'élément résistif.

17. Dispositif d'inhalation comportant l'ensemble selon l'une quelconque des revendications précédentes et comprenant en outre une pièce du corps principal (630) qui comporte une source d'alimentation destinée au dispositif et une unité de commande.
